# EUROPEAN PATENT SPECIFICATION

(11) **EP 0 501 442 B1**
(45) Date of publication and mention of the grant of the patent: **06.12.1995**
(21) Application number: 92103256.1
(22) Date of filing: 26.02.1992
(51) Int. Cl.: A61K 31/55

(54) **8-chloro-benzothiazepine-compound for prophylaxis and treatment of sequelae of cerebral neurocyte necrosis**
8-Chloro-benzothiazepin-Verbindung zur Prophylaxe und Behandlung der Folgen der zerebralen Nekrose von Neurozyten
8-Chloro-benzothiazépine-composé pour la prophylaxie et le traitement des conséquences de la Necrosie cérébrale des cellules nerveuses

(30) Priority: 26.02.1991 JP 117061/91
(43) Date of publication of application: 02.09.1992
(73) Proprietor: TANABE SEIYAKU CO., LTD., Chuo-ku, Osaka (JP)
(72) Inventor: Murata, Sakae, Kawagoe-shi, Saiatama-ken (JP)
(74) Representative: Hansen, Bernd, Dr. Dipl.-Chem.

(56) References cited:
- EP-A- 0 278 449
- WO-A-89/11281
- JOURNAL OF HYPERTENSION, (suppl.), vol. 6, no. 4, 1988; S. MURATA et al., S-759
- PHARMACOLOGIST, vol. 30, 1987; W. MORRONE et al., p. A-37
- JAPANESE JOURNAL OF PHARMACOLOGY, vol. 55, suppl. I, 1991, Proceedings of the 64th Annual Meeting, Kobe (JP), 24- 27 March 1991, The Japanese Pharmacological Society; R. YAMAUCHI et al., p. 609
- JOURNAL OF PHARMACOLOGICAL & EXPIMENTAL THERAPEUTICS, vol. 259, no. 1, October 1991, American Society for Pharmacology & Experimental Therapeutics, US; D. BLEAKMAN et al., pp. 430-438
- EUROPEAN JOURNAL OF PHARMACOLOGY, vol. 183, no. 3, 1990; S. MURATA et al., pp. 1070-1071

## Description

### BACKGROUND OF THE INVENTION

This invention relates to a prophylactic and curing agent for cerebral neurocyte necrosis comprising a benzothiazepine derivative as an active ingredient.

Cerebral neurocyte dyscrasia has been said to be one of the causes for symptoms such as dementia, parkinsonism, false bulbar paralysis, somnipathy, central pain, paraplegia in flexion and spasm. Thus, development of an agent which can prevent necrosis of cerebral neurocytes, and can prevent and cure these symptoms has been demanded strongly.

It has been known that 2-(4-methoxyphenyl)-3-lower alkanoyloxy-5-(2-(di-lower alkylamino)ethyl)-8-chloro-2,3-dihydro-1,5-benzothiazepin-4(5H)-one has actions on blood vessels and blood components such as cerebral and coronary vasodilating action, hypotensive action and platelet aggregation inhibitory action (Japanese Patent Publication No. 13994/1988). However, there has not been known about an action on cerebral neurocytes, which is not on a blood vessel system.

### SUMMARY OF THE INVENTION

The present inventors have found unexpectedly that the above benzothiazepine compound which has been known to have cerebral and coronary vasodilating action and hypotensive action has prophylactic and curing effect on cerebral neurocyte necrosis, and thus, the present invention is to provide the novel pharmaceutical composition.

The present invention relates to a pharmaceutical composition comprising a benzothiazepine derivative represented by the formula: 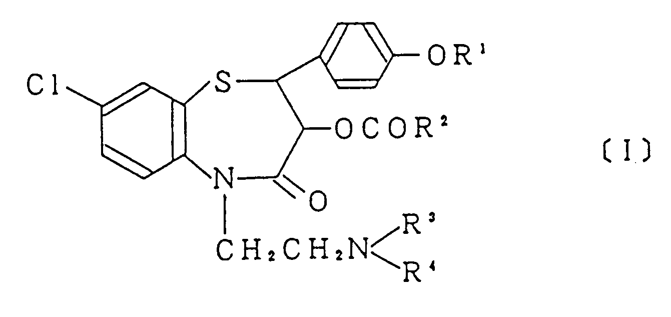
(wherein R¹ to R⁴ each represent a C₁-C₆ alkyl group) or a phamacologically acceptable salt thereof as an active ingredient.

In the pharmaceutical composition of the present invention, an active ingredient compound thereof acts directly on cerebral neurocytes and shows excellent preventive effect on necrosis of cerebral neurocytes, so that the pharmaceutical composition of the present invention is useful as a prophylactic and curing agent for cerebral neurocyte necrosis.

In the following, the present invention is explained in detail.

As a specific example of the active ingredient of the present invention, there may be mentioned a compound of the formula (I) in which R¹ to R⁴ are each an alkyl group having 1 to 6 carbon atoms.

The benzothiazepine derivative (I) which is the active ingredient of the present invention includes four kinds of optical isomers ((+)-cis, (-)-cis, (+)-trans and (-)-trans isomers) based on asymmetric carbon atoms at 2-position and 3-position of a benzothiazepine skeleton and a mixture thereof, and among them, a cys isomer is particularly preferred.

In the present invention, the benzothiazepine derivative (I) can be used in the free form or in the form of a pharmacologically acceptable salt thereof. As the pharmacologically acceptable salt, there may be used, for example, an inorganic acid addition salt such as hydrochloride, hydrobromide, hydroiodide, perchlorate, sulfate and phosphate; and an organic acid addition salt such as oxalate, maleate, fumarate, tartarate and methanesulfonate.

The dose of the benzothiazepine derivative (I) or a pharmacologically acceptable salt thereof varies depending on an administration method, and age, body weight and state of a patient, but, in general, it may be preferably about 0.05 to 20 mg/kg, particularly about 0.5 to 5 mg/kg per day.

The pharmaceutical composition of the present invention can be used orally or parenterally.

The administration form in the case of oral administration may be either a solid form such as a tablet, a powder, a capsule and a granule or a liquid form such as a solution and a suspension, which can be used as a medical preparation together with a medical carrier suitable for oral administration. As such a medical carrier, there may be used any conventional ones, for example, a binder (syrup, gum arabic, gelatin, sorbitol, tragacanth gum and polyvinyl pyrrolidone), an excipient (lactose, sugar, cornstarch, potassium phosphate, sorbitol and glycine), a lubricant (magnesium stearate, talc, polyethylene glycol and silica), a disintegrating agent (potato starch) and a humectant (sodium lauryl sulfate).

On the other hand, the administration form in the case of parenteral administration is preferably an injection or an dripping injection by using a distilled water for injection, a physiological saline solution or a glucose aqueous solution.

The benzothiazepine derivative (I) which is the active ingredient of the present invention can be prepared by, for example, the method disclosed in Japanese Patent Publication No. 13994/1988.

### EXAMPLES

The present invention is described in detail by referring to Example.

### Experiment example (Effect of preventing necrosis of hippocampal cells (in vitro))

Hippocampi were obtained from brains of SD strain fetal rats, and treated with trypsin for dissociation. The cell suspension was plated on previously cultured glia cells of brains from fetal rats in a medium of Dulbecco's modified Eagle's medium (Dulbecco, R. et al (1960), Virology, 12, p. 185) supplemented with 10 % heat-inactivated fetal bovine serum.

After seven to ten days in culture, the medium was removed and the cultures were exposed to 1 mM KCN in a physiological salt solution (composition (mM): NaCl, 145; KCl, 5; MgCl₂, 1; HEPES, 10; glucose, 10; pH, 7.4) with or without sample (10 »M). After a 30-minute exposure, cells were returned to the culture-conditioned medium and incubated for 24 hours. The survival rate of the neurons were estimated by comparing the number of neurons in one identical field before and 24 hours after exposure to KCN.

The results are shown in Table 1.

**Table 1**

| Sample | Survival rate of hippocampal cells*⁾ |
|---|---|
| (Compound of the invention) (+)-cis-2-(4-methoxyphenyl)-3-acetoxy-5-[2-(dimethylamino)ethyl]-8-chloro-2,3-dihydro-1,5-benzothiazepin-4(5H)-one·maleate | 75.4 % |
| Non-administered control group | 43.7 % |

| | |
|---|---|
| *) The survival rate of the group not treated with KCN and the sample is defined as 100 %. | |

From Table 1, it can be seen that the active ingredient of the present invention has excellent effect of preventing necrosis of hippocampal cells.

Since the benzothiazepine derivative (I) which is the active ingredient of the present invention or a pharmacologically acceptable salt thereof has action of curing and/or action of preventing cerebral neurocyte damages, it shows excellent effect of preventing necrosis of cerebral neurocytes. Thus, the pharmaceutical composition of the present invention can be effectively used for preventing, curing and/or alleviating various disorders involving cerebral neurocyte necrosis, for example, disturbances of consciousness (somnolence, stupor, clouding of consciousness and coma), motor paralyses (hemiplegia and parkinsonism), speech and language disorders (articulation disorders and aphasia), sense disorders (pain, numbness and heat), mental disorders (dementia, hallucination, delusion, delirium, violent behavior, depressed feeling, neurotic symptoms, poriomania and emotional incontinence), ophthalmic symptoms and dysuria, and further preventing recurrence and preventing exacerbation and progress of the above symptoms.

Further, the benzothiazepine derivative (I) or a pharmacologically acceptable salt thereof has low toxicity and high safety. For example, when 2-(4-methoxyphenyl)-3-acetoxy-5-[2-(dimethylamino)ethyl]-8-chloro-2,3-dihydro-1,5-benzothiazepin-4(5H)-one·maleate is orally administered to mice, its 50 % lethal dose (LD₅₀) was about 800 mg/kg.

## Claims

1. Use of a benzothiazepine compound represented by the formula: 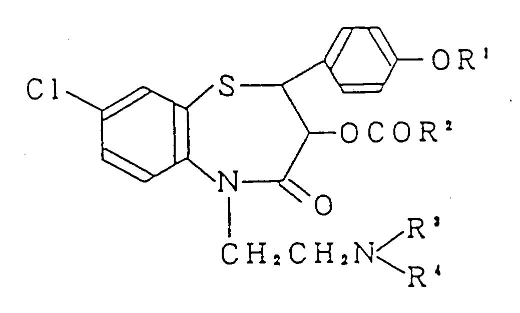 (wherein R¹ to R⁴ each represent a C₁₋₆ alkyl group) or a pharmacologically acceptable salt thereof for the preparation of a prophylactic and/or curing agent for cerebral neurocyte necrosis.

2. Use of a benzothiazepine compound according to claim 1 wherein R¹ to R⁴ are each methyl group.

3. Use of a benzothiazepine compound according to claim 1 wherein the compound is (+)-cis-2-(4-methoxyphenyl)-3-acetoxy-5-[2-(dimethylamino)ethyl]-8-chloro-2,3-dihydro-1,5-benzothiazepin-4(5H)-one·maleate.

## Patentansprüche

1. Verwendung einer Benzothiazepinverbindung der Formel: 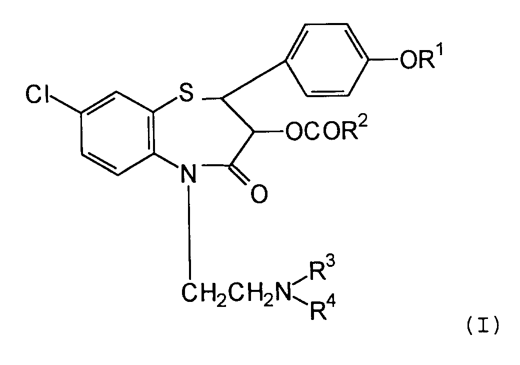 worin R¹ bis R⁴ jeweils eine C₁₋₆ -Alkylgruppe repräsentieren, oder ein pharmakologisch vertretbares Salz davon, zur Herstellung eines Prophylaxe- und/oder Heilmittels für cerebrale Neurocytennekrose.

2. Verwendung einer Benzothiazepinverbindung gemäss Anspruch 1, worin R¹ bis R⁴ jeweils eine Methylgruppe sind.

3. Verwendung einer Benzothiazepinverbindung gemäss Anspruch 1, worin die Verbindung (+)-cis-2-(4-Methoxyphenyl)-3-acetoxy-5-[2-(dimethylamino)ethyl]-8-chlor-2,3-dihydro-1,5-benzothiazepin-4-(5H)-on·maleat ist.

## Revendications

1. Utilisation d'un composé de benzothiazépine représenté par la formule: 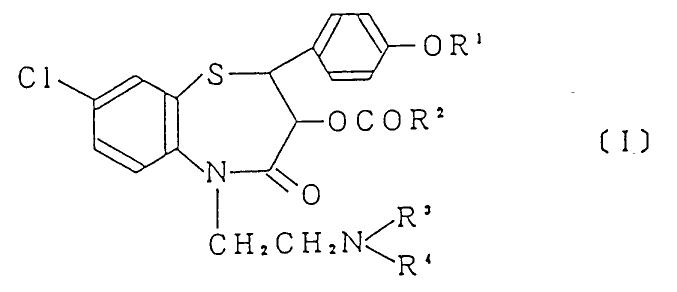 (dans laquelle R¹ à R⁴ représentent chacun un radical alkyle en C₁₋₆)
ou d'un sel pharmaceutiquement acceptable de celui-ci pour la préparation d'un agent pour la prophylaxie et/ou le traitement de la nécrose des neurocytes cérébraux.

2. Utilisation d'un composé de la benzothiazépine selon la revendication 1, dans lequel R¹ à R⁴ représentent chacun un radical méthyle.

3. Utilisation d'un composé de la benzothiazépine selon la revendication 1, dans lequel le composé est le maléate de (+)-cis-2-(4-méthoxyphényl)-3-acétoxy-5-[2-(diméthylamino)éthyl]-8-chloro-2,3-di-hydro-1,5-benzothiazépine-4(5H)-one.
